(19) Europäisches Patentamt
European Patent Office
Office européen des brevets

(11) Publication number: **0 383 403 A1**

# (12) EUROPEAN PATENT APPLICATION

(21) Application number: **90200335.9**

(22) Date of filing: **14.02.90**

(51) Int. Cl.[5]: **C12P 13/02, C12P 13/04, C12P 41/00, C12P 17/10**

The microorganism(s) has (have) been deposited with the National Collections of Industrial and Marine Bacteria Ltd. under numbers NCIB 40042, 40041, 40113.

(30) Priority: **16.02.89 EP 89200380**

(43) Date of publication of application: **22.08.90 Bulletin 90/34**

(84) Designated Contracting States: **BE CH DE DK ES FR GB IT LI NL**

(71) Applicant: **STAMICARBON B.V.**
**Mijnweg 1**
**NL-6167 AC Geleen(NL)**

Applicant: **NOVO-NORDISK A/S**
**Novo Allé**
**DK-2880 Bagsvaerd(DK)**

(72) Inventor: **Hermes, Hubertus Franciscus Maria**
**Overstraat 59**
**NL-6151 CM Sittard(NL)**
Inventor: **Peeters, Wijnand Peter Helena**
**Breetsepeelweg 2**
**NL-5993 NC Maasbree(NL)**
Inventor: **Peters, Peter Josephus Hubertus**
**Kochstraat 6**
**NL-6164 HB Geleen(NL)**

(74) Representative: **Hoogstraten, Willem Cornelis Roeland et al**
**OCTROOIBUREAU DSM Postbus 9**
**NL-6160 MA Geleen(NL)**

## (54) Process for preparation of organic chemicals.

(57) Process for the racemization of an amino acid amide with the general Formula I

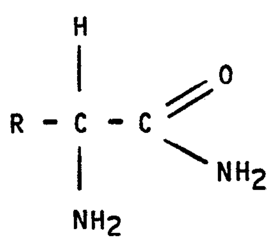

(I)

the amino acid amide being brought into contact with an enzyme with amino acid amide racemase activity towards the amide in question, which enzyme is present in species of the genus Klebsiella and related genera such as Enterobacter, Escherichia, Shigella, Citrobacter and Salmonella from the family of the Enterobactereareae. In particular the invention relates to a process for enzymatic preparation of optically active amino acids from amino acid amides by bringing the amino acid amide into contact with a biocatalyst which in addition possesses L- or D-amidase activity.

EP 0 383 403 A1

# PROCESS FOR PREPARATION OF ORGANIC CHEMICALS

The present invention relates to a process for complete or partial racemization of compounds of the general Formula I

R-CH($NH_2$)-$CONH_2$ (I)

wherein R represents indolyl, benzyloxy, lower alkyl optionally substituted by hydroxy, mercapto, amino, halogen, phenyl, phenoxy, benzyl or lower alkylthio, or R represents phenyl optionally substituted by one or more of the following substituents: hydroxy, amino, halogen, carboxy or lower alkoxy.

## BACKGROUND OF THE INVENTION

Amino acid amides are readily obtainable compounds by chemical as well as by enzymatical processes, and are as such important as precursors in the production of amino acids. Optically active amino acids can be obtained by e.g. enantioselective enzymatic hydrolysis of amino acid amides. Simultaneous enzymatical racemization of the amino acid amides, which is thusfar unknown, will be of great advantage beçause then complete conversion of the amino acid amide into the desired optically active amino acid is possible.

As used in this specification, a stereoselective (enantioselective enzyme is one that reacts exclusively or preferentially with one of two enantiomers, thereby producing an optically pure or optically active product, respectively. A stereospecific (enantiospecific) enzyme is one that reacts exclusively with one of the enantiomers to produce an optically pure product.

Optically active α-H,-amino acids constitute a class of organic compounds of great industrial importance. Two major groups of these compounds can be identified: the natural amino acids found in nature e.g. as constituents of proteins, peptides and a wide variety of other important compounds, and the so-called unnatural amino acids which share certain characteristic structural features with the naturally occurring amino acids but which are not natural constituents of living matter. Both groups of amino acids find important industrial applications. The naturally occurring amino acids are thus applied abundantly as food and feed additives and in the pharmaceutical industry e.g. as components of infusion liquids. In recent years, also unnatural amino acids have found extensive uses for example as intermediates for various pharmacological compounds.

Due to their molecular structure amino acids of Formula I can occur in two distinct forms differing in respect to the so-called chirality of the amino acid molecules. These two forms of a given amino acid are usually denoted as the D- or the L-form of the amino acid. Most amino acids found in nature are of the L-configuration and it has turned out to be essential that amino acids used for food and feed additives are of this configuration since the corresponding Disomers cannot, usually, be metabolised by living cells and will even interfere with normal cell metabolism and function. In consequence of this, these unnatural amino acids can often be used as building blocks for pharmaceutical and agrochemical products. For example, D-phenylglycine and D-p-hydroxyphenylglycine are used for broad spectrum antibiotics such as Ampicillin and Amoxycillin, respectively.

Because of the growing demand for selective products in the pharmaceutical, food and agrochemical industries it is highly desirable to have available methods for producing optically pure, i.e. enantiomerically pure, amino acids of the D- as well as of the L-configuration while, on the contrary, mixtures of the two enantiomeric forms of a given amino acid, which are denoted racemates when the two forms are present in equal quantities, are of a more limited industrial interest. The reason for this is that only one of the two optical isomers exhibits a clear activity in the desired applications. The other optical isomer, however, may exhibit no activity, hardly any activity, or even undesired side activity.

Known processes for preparing optically active compounds are e.g. fermentation, asymmetrical synthesis and chemical or enzymatic resolution processes. They have been described in reviews, e.g. by G. Schmidt-Kastner and P. Egerer in Biotechnology, H.-J. Rehm and G. Reed (Eds.), Verlag Chemie, Florida-Basel, Vol. 6a, pp. 387-421 (1984) and by E.M. Meijer et al. in Symposium Proceedings: "Biocatalysts in Orga nic Syntheses", Noordwijkerhout, April 14-17, 1985, Elsevier Science Publishers.

For the production of L-amino acids, in particular microbial fermentation is a suitable preparation method, besides a number of enzymatic resolution methods and enzymatic asymmetrical syntheses. Thus, today an increasing number of natural amino acids are produced by fermentation techniques which, however, cannot be applied for production of unnatural amino acids, whether these are D-forms of the naturally occurring L-amino acids (with exception of a few naturally occurring D-amino acids, e.g. D-alanine), or amino acids which by virtue of their chemical composition (depending on the R-substituent) do not occur in nature.

For the preparation of these unnatural amino acids the above-mentioned processes such as asymmetrical synthesis and chemical or enzymatical resolution can be used.

In recent years there has been an increasing interest in applying enzymes in organic synthesis in general. The developments in this area are due to the recognition that enzymes, although occurring and applied in nature as biocatalysts for synthesis of naturally occurring compounds, do catalyze a variety of reactions which usually do not take place in nature but which can nevertheless be utilised to advantage by the organic chemist. These developments regarding the use of enzymes for synthesis are of particular interest in connection with amino acid production since enzymes, being enantioselective, possess the ability to catalyze formation of optically pure compounds. In such processes the enantioselective properties of enzymes are utilized to generate an optically pure compound by converting only one of the enantiomers present in a racemic mixture typically made available by chemical synthesis.

Besides of the high stereoselectivity, enzymatic processes are preferred in view of the mild reaction conditions and the usually broad substrate specificity. The last-mentioned aspect refers to the fact that one and the same enzyme preparation can be used for the preparation of several different amino acids.

An excellent example of an enzymatic resolution method is described in US-A-3,971,700, US-A-4,080,259 and US-A-4,172,846. In these patents a racemic D,L-amino acid amide is brought into contact with a preparation containing L-enantioselective amidase, as indicated in Scheme I.

Scheme I.

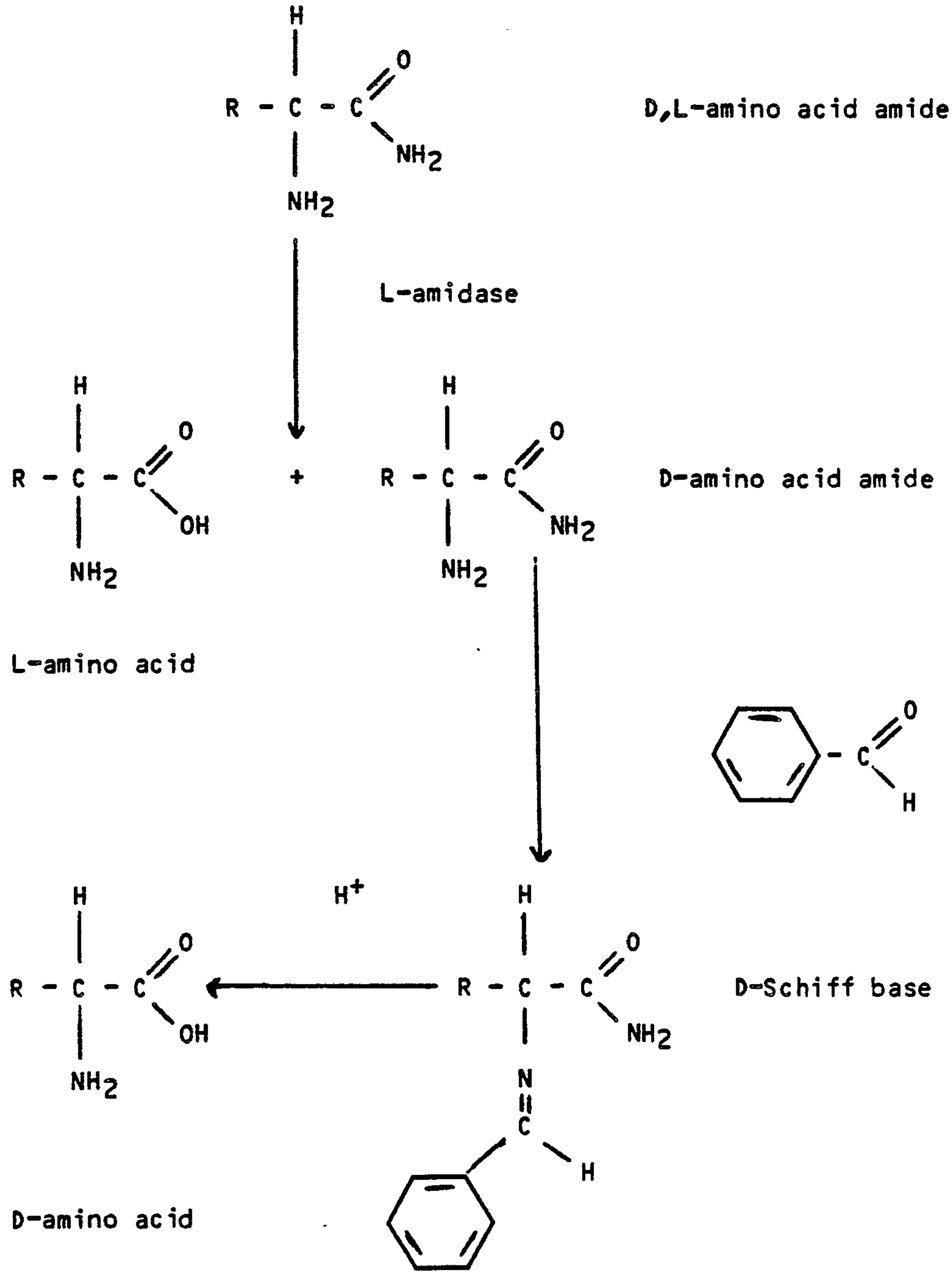


Only the L-amino acid amide is hydrolyzed, and a mixture of L-amino acid and D-amino acid amide is formed. The L-amino acid can be recovered therefrom by several methods, e.g. by crystallization, by extraction or by ion exchange chromatography. Also, the D-amino acid amide can be precipitated as a Schiff base by reaction with e.g. benzaldehyde. After recovery of the Schiff base, the D-amino acid amide can be recovered and from this the D-amino acid can subsequently be recovered by chemical (acid) hydrolysis as well as by enzymatic hydrolysis (see e.g. EP-A-179,523).

The method disclosed in the above patents serves, therefore, as a means for preparation of optically pure D- as well as L-amino acids. However, it remains a disadvantage that at most 50% of the racemic starting material can be utilized for the recovery of the corresponding D- or L-amino acid.

As seen, amides are important precursors for the production of optically active amino acids.

The general principle of utilising enantioselective properties of enzymes to separate a racemic mixture of a precursor for an amino acid into a mixture of the optically pure amino acid and the optically pure precursor has a wide applicability but suffers from at least one important disadvantage: usually only one

form of the optically pure amino acid is required in large quantities, there being no need to produce its enantiomeric counterpart. Accordingly, there is usually no need for either the optically pure precursor or the optically active amino acid remaining after production of the amino acid actually needed has been carried out. In industrial practice it is required, therefore, to racemize and recycle unused material when applying the principle of enantioselective enzymatic synthesis for production of organic chemicals. In many instances racemization of precursor molecules remaining after conversion of a racemic mixture of the precursor is thus an integral part of enzymatic synthesis of optically pure amino acids. Accordingly, quite some attention has been devoted to this problem there being available a number of chemical methods for racemization of amino acid precursors.

For example, D-amino acid amides have been found to be readily racemizable by conversion into their D-N-benzylidene derivatives (see US-A-4,094,904 and US-A-4,172,846).

Chemical methods for racemization of amino acid amides are, however, usually not compatible with enzymatic reactions, one consequence being that the racemization and resolution steps have to be carried out separately. Moreover, chemical methods for racemization of amino acid amide precursors suffer from the disadvantage that they impose an extra cost onto the overall process due to the cost of chemicals, equipment, losses during the racemization step by e.g. by-product formation and a variety of other circumstances.

## STATEMENT OF THE INVENTION

Surprisingly, we have discovered the existence of enzymes with amino acid amide racemase activity, which was previously unknown, in species of the genus Klebsiella and related genera. Accordingly, the invention provides a process for racemization of amino acid amides of the general Formula I characterized by exposing the amides to an enzyme with amino acid amide racemase activity towards the amide in question, which enzyme is present in species of the genus Klebsiella and related genera such as Enterobacter, Escherichia, Shigella, Citrobacter and Salmonella from the family of the Enterobactereaceae.

Numerous advantages can be gained by applying the process of the invention for racemization of amino acid amides instead of using chemical or other technologies for this conversion. First of all, the mild conditions usually prevailing during enzymatic reactions result in low losses of material through by-product formation. Also, the process of the invention can be applied without any use of costly chemicals which after their use may even give rise to environmental problems e.g. in regard to their disposal. Further, the conditions used in the process of the invention make it possible to combine enzymatic racemization of amino acid amide with enantioselective enzymatic hydrolysis of the D- or L-amide to prepare optically active amino acid in one single step without the need to separate and recycle the unreacted amide.

The process according to the method of the invention for complete or partial racemization of compounds of the general Formula I

$R\text{-}CH(NH_2)\text{-}CONH_2$ (I)

wherein R represents indolyl, benzyloxy, lower alkyl optionally substituted with hydroxy, mercapto, amino, halogen, phenyl, phenoxy, benzyl or lower alkylthio, or R represents phenyl optionally substituted by one or more of the following substituents: hydroxy, amino, halogen, carboxy or lower alkoxy, is characterized by exposing said compounds to an enzyme with amino acid amide racemase activity towards the amide in question, which enzyme is present in species of the the genus Klebsiella and related genera such as Enterobacter, Escherichia, Shigella, Citrobacter and Salmonella from the family of the Enterobactereaceae.

Of course, the process of the invention can also be applied to amides of heterocyclic amino acids, where the group R in Formula I is also linked to the α-amino group. One example is the natural amino acid proline.

Examples of the group R are as follows: methyl, isopropyl, secondary butyl, phenyl, p-hydroxyphenyl, benzyl, 2-phenyl-ethyl, 1-hydroxyethyl, mercaptomethyl, methylthiomethyl and phenoxymethyl. Furthermore, the phenyl, indolyl and benzyl group may be substituted by one of the following groups: hydroxy, amino, halogen, carboxy, and lower alkoxy. The term "lower alkyl" designates alkyl containing less than 8, preferably less than 5, carbon atoms. Similarly, lower alkoxy contains less than 8, preferably less than 5, carbon atoms.

In a particularly advantageous embodiment, the process of the invention is used to prepare an optically active amino acid from a corresponding amide by simultaneously contacting the amide with amino acid amide racemase and stereoselective D- or L-amidase with activity towards the D- or L-amino acid amide in question. (This amidase enzyme is also sometimes called aminopeptidase). The reactions are shown in Scheme II.

Scheme II

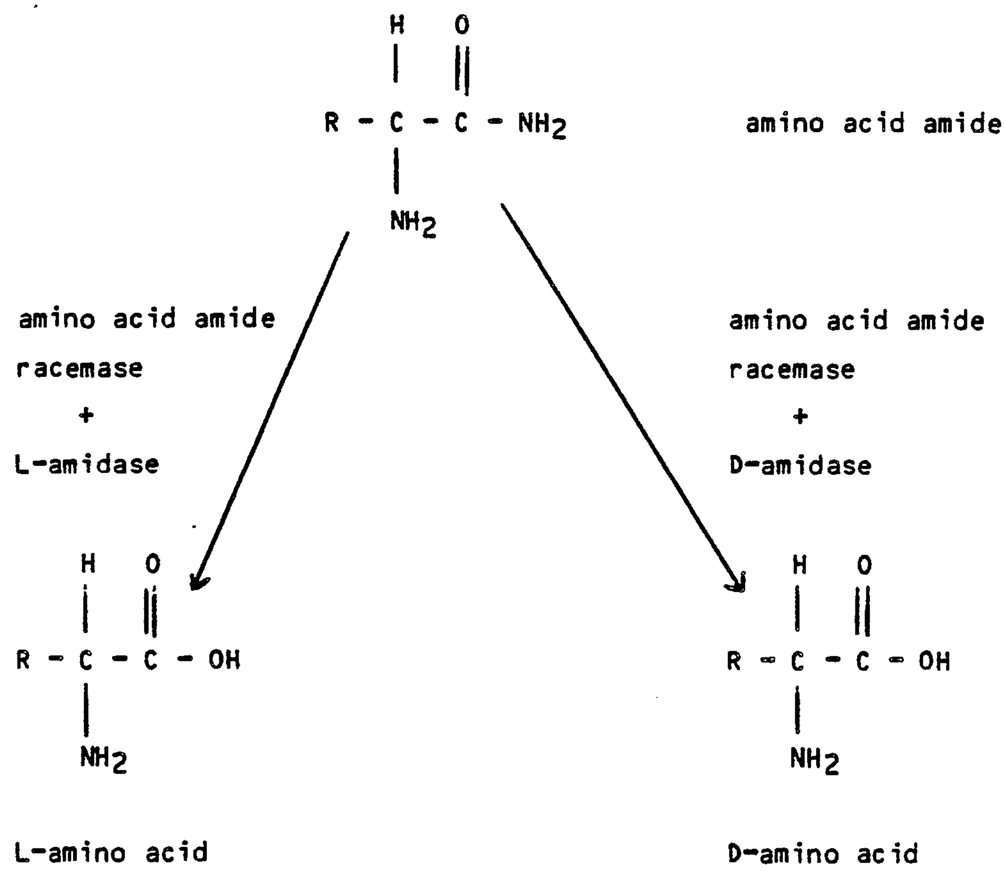


If L-amidase is used, D-amidase should be absent, and vice versa. Further, amino acid racemase with activity toward the amino acid in question should be absent.

The amino acid amide can be supplied in pure D- or L-form or as a mixture of the two, e.g. a racemic mixture. With this process, it is even possible to convert D-amino acid amide into L-amino acid, or L-amide into D-acid. It may be particularly convenient to use a racemic mixture of amides, prepared chemically.

The advantage of this process is that from mixtures of enantiomers, including racemic mixtures, of an amino acid amide, as well as from optically pure D- or L-amino acid amide, an optically pure D-or L-amino acid can be prepared stereoselectively, the amino acid amide being fully converted to the desired amino acid.

The enzymatic process according to the invention may be car ried out, for example, in a batch-wise fashion by stirring a mixture of the amino acid amide to be racemized and the enzyme in an aqueous medium under control of the pH value and the temperature of the reaction mixture. The pH value of the reaction mixture can vary between 6 and 12, in particular between 7.5 and 9.5. The reaction temperature may be between the freezing point of the reaction medium and about 65¤C, preferably between 20 and 45¤C, and most preferably about 37¤C. Outside these ranges the activity and/or the stability of the biocatalyst is generally insufficient for a satisfactory yield. If required, organic solvents may be added to the reaction mixture to increase the solubility of the reactants such solvents being, for example, alcohols such as ethanol, methanol, isopropanol or t-butanol, or other organic solvents like dioxane, N,N-dimethylformamide, dimethylsulfoxide or hexamethylphosphorous triamide. The reaction may also be carried out in a two-phase system using a suspension of reactants or two immiscible solvents like, for example, water and a hydrocarbon like hexane or cyclohexane, or in a fully organic phase of a hydrophobic, water-saturated, organic solvent.

The racemases applied in the process according to the invention may be purified enzymes, a crude enzyme solution, microbial cells exhibiting the required activity, a homogenate of cells or permeabilized cells. If required, the enzyme may be applied in an immobilized state or in a chemically modified form to ensure a good stability and reactivity of the enzyme under the conditions utilized. The process of the

invention may further be carried out concomitantly with enantioselective, enzymatic hydrolysis of amino acid amides into amino acids. The abovementioned enzymes or enzyme activities can also be denoted as biocatalyst(s). This is obviously understood to include a mixture of biocatalysts each containing one of the enzyme activities to be combined, or preferably one biocatalyst in which both enzyme activities are present. The reaction mixture can also be passed through alternating reactors containing the amino acid amide amidase and the racemase enzyme activity, respectively.

Enzymes applied in the process of the invention are enzymes catalyzing conversion of one enantiomer of an amino acid amide into its antipode (or, in case the amide contains additional asymmetric centers, into the epimeric compound).

It is particularly convenient to test enzymes to be applied according to the method of the invention by employing analytical methods such as chiral thin layer chromatography (TLC) as e.g. described in J. Chromatogr. 330 (1985), p. 375-378 by Brinkman et al. or chiral high pressure liquid chromatography (HPLC) as e.g. described in Anal. Biochemistry, 121, 370 (1982) by Weinstein et. al.

The enzymes applied according to the method of the invention may be isolated from bacterial species of the family Enterobactereaceae, such as those belonging to the related genera Enterobacter (e.g. E. sazakii, E.cloacae or E.aerogenes), Escherichia (e.g. E.coli), Shigella (e.g. S.boydii, S.flexneri), Klebsiella - (e.g. K.oxytoca, K.ozaenae, or K.rhinoscleromatis), Citrobacter (e.g. C.freundii, or C.diversus), and Salmonella (e.g. S.arizonae). In particular, the amino acid amide racemase enzyme may be obtained from strains of Klebsiella especially Klebsiella oxytoca . The racemase enzyme from the following strain, which has been deposited by the applicants for patenting purposes under the terms of the Budapest Treaty with the National Collections of Industrial & Marine Bacteria Ltd is especially suited for the process according to the invention:

Deposit No.: NCIB 40113
Deposit date: 8 February 1989
Depositor: NOVO
Depositor's reference: C1 (ID 2045)
Taxonomic designation: Klebsiella oxytoca.

The amino acid amide racemase used in the invention is preferably derived from a strain of one of the abovementioned microoorganisms, and it can be obtained e.g. by cultivation of the strain in a suitable medium or by cultivation of a transformed host organism containing a gene encoding for and expressing this activity, the gene having been obtained from one of the above organisms.

The suitability of a given strain as a source of amino acidamide racemase or the presence of such an enzyme in crude enzyme preparations is conveniently tested by incubating cells of the microorga nism in question or the crude enzyme with the optically pure forms of the amino acid amide of interest. During the incubation aliquots of the reaction mixture are taken and analyzed for the content of amino acid amides and amino acids by employing e.g. chiral TLC or chiral HPLC. The occurrence of an amino acid amide racemase will, in those cases where the incubation is performed using the L-amino acid amide, give rise to the presence of the corresponding D-amino acid amide and/or the D-amino acid (when the racemase activity is used concomitantly with a D-amidase activity . Conversely, the L-amino acid amide and/or the L-amino acid (when using concomitantly an L-amidase) will be generated if the D-amino acid amide is incubated with cells or enzymes exhibiting racemase activity.

When carrying out the abovementioned tests for the presence of an amino acid amide racemase in microorganisms or crude enzymes, amino acid racemases and D- and/or L-amidases will often be encountered and make the interpretation of the experimental results difficult. However, by testing directly for the presence of these amino acid racemases and their activities by incubating optically pure amino acids with the crude enzymes or cells in question some of the difficulties are readily overcome. Alternatively, amino acid racemase inhibitors as e.g. β-chloro-L-alanine may be added to the incubation mixture. Another problem with respect to the interpretation of the experimental results is the presence of D- and/or L-amidase activity simultaneously with the amino acid amide racemase activity. Amino acid amide amidase inhibitors such as α-amino methyl ketones as described by Jahreis et. al. in Biomed. Biochem. Acta. 46, 683 (1987) may be employed to prevent conversion of the amino acid amides to the corresponding amino acids.

Finally, in those instances where analysis of cells or crude enzymes indicates the presence of amino acid amide racemase activity the crude enzymes or the enzymes from the cells tested may be subjected to purification using the methods for protein and enzyme purification well described in the art. Fractions obtained during such purification procedures can be analyzed for amino acid amide racemase activity by employing a chiral TLC- or chiral HPLC-system.

Microorganisms to be tested for amino acid amide racemase activity may be furnished in a number of independent ways:

1. The microorganisms may be obtained from culture collections. Before test of such organisms it is preferred to expose or grow the microorganism in the presence of e.g. the D-amino acid amide in question. Next to the possibility of induction of D-amidase activity also amino acid amide racemase activity may be induced.

2. The microorganisms may be selected among strains known to produce amino acids of the unnatural configuration, peptides containing amino acids of the unnatural configuration, or compounds derived from amino acids of unnatural configurations. Such microorganisms are listed e.g. in Handbook of Microbiology published by CRC Press Ltd. or in related works.

3. Microorganisms that may produce amino acid amide racemases may also be obtained by exposing an available microorganism to a mutagenic agent such as e.g. NTG, ultra violet radiation, or gamma radiation. Such treatments are known in the art to alter the specificity of enzymes and to induce expression of enzymes from cryptic genes.

4. Microorganisms producing amino acid amide racemases may also be obtained directly by screening employing e.g. enrichment culture techniques. A preferred enrichment method involved addition of environmental soil samples to a suitable enrichment medium supplemented e.g. with the pure D-amino acid amide in question as the sole carbon or nitrogen source. Using this procedure microorganisms producing amino acid amide racemases will be enriched and they can subsequently be isolated by conventional microbiological techniques. A selection pressure can be established during the screening by employing defined and semisynthetic media. The microorganisms isolated are assayed by employing the methods described above. It is preferred to perform the enrichment under aerobic or anaerobic conditions as well as at different temperatures and pH-values in order to obtain a collection of amino acid amide racemases suitable for use under different conditions.

For the purpose of further elucidation it is indicated here that the strain NCIB 40113 (Klebsiella oxytoca) which contains an enzyme with amino acid racemase activity was isolated from an enrichment experiment, while performing cultivation in YCB (Yeast Carbon Base, Difco$^R$) complex medium (10 g/l) with D-phenylglycine amide as only N-source at a temperature of 30¤C and a pH of 7. As will be shown in the Examples this strain NCIB 40113 also contains D-amidase activity.

An illustration of how a strain with D-amidase activity can be furnished is the isolation of the strain NCIB 40041: Erlenmeyer flasks containing 50 ml of an enrichment medium devoid of nitrogen sources but supplemented with D-amide are inoculated with soil samples. After seven days flasks showing good growth are selected and subcultivated in the same medium. After another seven days flasks showing good growth are selected and their content is analyzed for the presence of D-amide. Those cultures devoid of D-amide are spread on agar plates from which colonies are selected and analyzed for amidase activity.

The strain NCIB 40041 was isolated by this procedure. This strain NCIB 40041, which has been designated as a Rhodococcus sp., was deposited for patenting purposes on 2 August, 1988 under the terms of the Budapest Treaty with the National Collections of Industrial & Marine Bacteria Ltd. by NOVO. Other D-amidases can be isolated in a similar fashion and their specificity may be influenced by selecting the applied D-amino acid amide in accordance with the need.

D-amidase may also be obtained (and brought to expression in the same way) from a strain of Pseudomonas putida NCIB 40042, which has been deposited for patenting purposes on 3 August, 1988 under the terms of the Budapest Treaty with the National Collection of Industrial & Marine Bacteria Ltd by DSM.

In particular, the combination of a biocatalyst with D-amid activity and a biocatalyst with a comparably high degree of amino acid amide racemase activity, and preferably one single microorganism expressing both activities to comparable degrees, offers a suitable method of preparing optically pure D-amino acids starting from D- or L-amino acid amides. The same holds, mutatis mutandis, for the combination of an L-amidase and an amino acid amide racemase, preferably expressed in one microorganism with comparably high activities, with regard to the preparation of optically pure L-amino acids from the abovementioned raw materials.

An example of a microorganism expressing both amino acid amide racemase and amidase activity, is the abovementioned strain of Klebsiella oxytoca NCIB 40113.

The inducible D-amidase activity is brought to expression by inoculating this strain in a culture medium in which a D-amino acid amide, preferably D-phenylglycine amide (whether or not as N-source) is present.

The expression of amino acid amide racemase activity together with L-amidase activity can be obtained by cultivating this Klebsiella oxytoca strain in the absence of a D-amino acid amide, as a result of which the D-amidase activity is not brought to expression.

The expression of amino acid amide racemase activity and D-amidase activity may possibly be obtained by, for example, preparing an L-amidase-negative mutant from the Klebsiella oxytoca strain using

classical genetic techniques (such as UV-irradiation, or alkylating reagents) or by using recombinant DNA-techniques, and then cultivating it in the presence of a D-amino acid amide (whether or not as N-source). It is also possible to obtain, by enzyme purification, purified fractions with D-amidase activity on the one hand and amino acid amide racemase activity on the other. Combination of these fractions then yields a preferred biocatalyst for the preparation of optically active D-amino acids.

Many microbial L-amidases are known and can be used in the practice of the invention. Besides the before mentioned enzyme from Klebsiella oxytoca strain NCIB 40113, an excellent example is the enzyme from Pseudomonas putida strain ATCC 12633.

The amidase activities can be derived from the indicated microorganisms in the same way as mentioned for the amide racemase.

The following examples are given for the purpose of illustrating the operation of the process of the invention without, of course, limiting in any manner to the examples:

Example I

The Klebsiella oxytoca strain NCIB 40113 was cultivated for 18 hours at 30¤C in respectively

a) a complex medium, which will be called here P-medium, consisting of nitrilotriacetic acid (0.2 g/l); $MgSO_4$ (0.6 g/l); $CaCl_2$ (0.1 g/l); ammonium molybdate (0.2 g/l); $KH_2PO_4$ (3.4 g/l); $Na_2HPO_4$ (4.45 g/l); yeast extract (Difco$^R$; 10 g/l); $FeSO_4$ (2 mg/l), and having a pH of 7.0; the cultivation medium was sterilised before inoculation during 15 minutes at 120¤C;

b) under the same conditions, in said P-medium which was supplemented with 0.2% (w/v) D-phenylglycine amide;

c) again under the same conditions YCB medium (Yeast Carbon Base, Difco$^R$; 10 g/l) which was supplemented with 0.2% (w/v) D-phenylglycine amide.

After harvesting of the cells by centrifugation and washing, each time 0.1 g of cell sludge was added to 5 ml of a 1% (w/v) solution of D-valine amide.

The reactions were conducted for 17 hours in a small vessel at 38¤C and pH 8.1, with shaking. Analysis of the reaction mixtures after incubation was performed by chiral TLC analysis, from which the formation of D- and/or L-valine could be seen. The estimated quantities are indicated below, in relative proportions, by +'ses.

| Cultivation medium | product formation | |
|---|---|---|
| | L-valine | D-valine |
| a) P | + | none |
| b) P + D-PG-amide | + + + | + + |
| c) YCB + D-PG-amide | + + + | + + |

These results show that the D- amidase activity towards valine amide is only brought to expression if the cultivation medium contains D-phenylglycine amide. The cells cultivated in medium a), i.e. without D-phenylglycine amide, posses (constitutive) L-amidase activity together with amino acid amide racemase activity. There is no amino acid racemase activity, as this would inevitably have led to the formation of both D- and L-valine in all assays.

Example II

In the same way as in Example I the Klebsiella oxytoca strain NCIB 40113 was cultivated in medium a) - c), and after harvesting of the cells by centrifugation and washing each time 0.1 g of cell sludge was added to 5 ml of a 1% (w/v) solution of D-leucine amide. The reactions were conducted for 17 hours in a small vessel at 38¤C and pH 8.1, with shaking.

The reaction mixtures after incubation were analyzed by chiral TLC methods, giving the following results.

| Cultivation medium | Product formation | |
|---|---|---|
| | L-leucine | D-leucine |
| a) P | + + + + | none |
| b) P + D-PG-amide | + + + | + + + |
| c) YCB + D-PG-amide | + + + + | + + + + |

Example III

In the same way as in the previous Examples cultivated cells from the Klebsiella oxytoca strain NCIB 40113 were harvested and washed. Each time 0.1 g of cell sludge was added to 5 ml of a 1% (w/v) solution of D-phenylglycine amide. The reactions were conducted for 17 hours in a small vessel at 38°C and pH 8.1, with shaking. Chiral TLC analysis of the reaction mixtures after incubation gave the following results.

| Cultivation medium | Product formation | |
|---|---|---|
| | L-phenylglycine | D-phenylglycine |
| a) P | + | - |
| b) P + D-PG-amide | + | + + + + |
| c) YCB + D-PG-amide | + | + + + + |

From the above experiments it may be concluded that both aromatic and aliphatic amino acid amides can be racemized enzymatically by exposing such amides to an enzyme preparation from Klebsiella oxytoca strain NCIB 40113, which also possesses D-amidase activity towards these amino acid amides.

## Claims

1. Process for complete or partial racemization of compounds of the general Formula I
$R\text{-}CH(NH_2)\text{-}CONH_2$ (I)
wherein R represents indolyl, benzyloxy, lower alkyl, optionally substituted by hydroxy, mercapto, amino, halogen, phenyl, phenoxy, benzyl or lower alkylthio, or R represents phenyl optionally substituted by one or more of the following substituents: hydroxy, amino, halogen, carboxy or lower alkoxy characterized by exposing said compounds to an enzyme with amino acid amide racemase activity towards the amide in question, which enzyme is present in species of the genus Klebsiella and related genera, such as Enterobacter, Escherichia, Shigella, Citrobacter and Salmonella from the family of the Enterobactereaceae.

2. Process according to claim 1, characterized by said enzyme being derived from a strain of Klebsiella, especially Klebsiella oxytoca.

3. Process according to claim 1 or 2, characterized by said enzyme being derived of Klebsiella oxytoca strain, NCIB 40113.

4. Process according to claim 1, characterized by said enzyme being obtainable from strain NCIB 40113.

5. Process according to any of the preceding claims characterized in that the racemization is carried out in the presence of an enantioselective amino acid amide amidase with activity towards the amide in question.

6. Process according to claim 5 characterized by said enantioselective amidase being L-specific.

7. Process according to claim 6 characterized by said L-specific amidase being derived from a strain of Klebsiella, preferably Klebsiella oxytoca, most preferably strain NCIB 40113, or from a strain of Pseudomonas, preferably Ps. putida, most preferably strain ATCC 12633 or strain NCIB 40042.

8. Process according to claim 5 characterized by said enantioselective amidase being D-specific.

9. Process according to claim 8 characterized by the D-specific amidase being derived from a strain of Klebsiella, preferably Klebsiella oxytoca, most preferably strain NCIB 40113, or being derived from a strain

of Rhodococcus sp., preferably the strain NCIB 40041, or being derived from a strain of Pseudomonas, preferably Ps. putida, most preferably strain NCIB 40042.

10 Process according to claim 5 characterized by the amide racemase and the enantioselective amidase acitivity being both expressed in one microorganism.

11. Process according to any of the preceding claims characterized by the process being carried out in the presence of an organic solvent.

European Patent Office

# EUROPEAN SEARCH REPORT

Application Number

EP 90 20 0335

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 199 407 (STAMICARBON)<br>* Claims * | 1 | C 12 P 13/02<br>C 12 P 13/04<br>C 12 P 41/00<br>C 12 P 17/10 |
| A | EP-A-0 179 523 (STAMICARBON)<br>* Claims * | 1,5 | |
| A | EP-A-0 181 675 (STAMICARBON)<br>* Claims * | 1,5 | |
| E | EP-A-0 307 023 (NOVO INDUSTRI; STAMICARBON)<br>* Claims; pages 8-9 * | 1-11 | |
| | | | TECHNICAL FIELDS SEARCHED (Int. Cl.5) |
| | | | C 12 P |

The present search report has been drawn up for all claims

| Place of search | Date of completion of the search | Examiner |
|---|---|---|
| THE HAGUE | 14-05-1990 | DELANGHE L.L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)